# EUROPEAN PATENT APPLICATION

(11) **EP 3 067 427 A1**
(43) Date of publication of application: **14.09.2016**
(21) Application number: 14860363.2
(22) Date of filing: 06.11.2014
(51) Int. Cl.: C12P 19/14

(54) **PRODUCTION METHOD FOR FRUCTOOLIGOSACCHARIDE**

(30) Priority: 06.11.2013 JP 2013230138
(71) Applicant: Meiji Co., Ltd., Koto-ku Tokyo 136-8908 (JP)
(72) Inventor: TOYOTA Kenji, Odawara-shi Kanagawa 250-0862 (JP); OHARA Hiroki, Odawara-shi Kanagawa 250-0862 (JP)
(74) Representative: Regimbeau
(86) International application number: PCT/JP2014/079450
(87) International publication number: WO 2015/068764

(57) **Abstract**

Provided is a method for producing fructooligosaccharide, wherein, in a reaction where fructooligosaccharide is produced by reacting β-fructofuranosidase with sucrose, and in a reaction step where glucose by-produced by the above reaction is decreased by glucose oxidase, an amount of dissolved oxygen is adjusted to 2 to 8 ppm. This method enables fructooligosaccharides to be produced inexpensively and efficiently.

## Description

### CROSS-REFERENCE TO RELATED APPLICATIONS

The present patent application claims priority based on Japanese Patent Application No. 2013-230138 (filed on November 6, 2013), which is an earlier filed patent application in Japan. The contents of this earlier patent application are incorporated herein by reference in their entirety as a part of the present specification.

### TECHNICAL FIELD

The present invention relates to a method for producing fructooligosaccharide. More specifically, the present invention relates to a method for preparing a sugar solution having a high content of fructooligosaccharide inexpensively and efficiently.

### BACKGROUND ART

Fructooligosaccharide (FOS) refers to oligosaccharides in which one or more fructose residues are bound to sucrose (GF) through β2-1 bonds. For example, sucrose to which one, two, and three fructose residues are bound are 1-kestose (GF2), nystose (GF3), and fructosylnystose (GF4), respectively. In general, commercially available fructooligosaccharide products contain 1-kestose, nystose, and fructosylnystose as main components.

The fructooligosaccharide has useful properties such as non-cariogenicity, the effect of promoting Bifidobacterium growth, the effect of promoting mineral absorption, and the like.

Therefore, there have been proposed methods for efficiently producing fructooligosaccharide of high purity (Patent Documents 1 to 4).

According to a conventional method for producing fructooligosaccharide by reacting fructofuranosidase with sucrose, a content ratio of fructooligosaccharide in a reaction mixture obtained by an enzymatic reaction is generally about 40 to 60%. Therefore, in order to separate the fructooligosaccharide and a specific oligosaccharides component efficiently from the reaction mixture, a necessity arises to further carry out a separation and purification means such as chromatographic separation and the like additionally (Patent Documents 3 and 4). When chromatographic separation is introduced, not only a cost of installing a dedicated facility is generated but also the production process becomes complicated.

On the other hand, use of a mixture system of fructofuranosidase and glucose oxidase has been proposed for the purpose of improving efficiency of the enzymatic reaction by fructofuranosidase. A content ratio of fructooligosaccharide in a reaction mixture obtained by the enzyme mixture system improved to at most 86% (Patent Documents 1 and 2).

### BACKGROUND ART DOCUMENTS

### Patent Document

Patent Document 1: Japanese Patent Laid-Open Publication No. 2010-273580
Patent Document 2: Japanese Patent Laid-Open Publication No. S62-14792
Patent Document 3: Japanese Patent Laid-Open Publication No. 2000-232878
Patent Document 4: WO 97/21718

### SUMMARY OF THE INVENTION

However, in the market, a supply of more economical fructooligosaccharide is desired in order to expand use of the fructooligosaccharide having excellent physiological functions. For that purpose, a method for producing fructooligosaccharide more efficiently in an industrial scale is needed. It is therefore a purpose of the present invention to provide a method for producing fructooligosaccharide inexpensively and efficiently.

The present inventors have found that, in a reaction process of reacting fructofuranosidase and glucose oxidase with sucrose as a substrate, a sugar solution having a content ratio of fructooligosaccharide of 90% or more can be obtained rapidly by maintaining a dissolved oxygen concentration in the enzymatic reaction mixture at 2 ppm or more. Furthermore, the present inventors have found that, even when the process is scaled up to a level which enables industrial production, production efficiency of the fructooligosaccharide can be maintained by introducing a fine air bubble generating device as a means for maintaining the dissolved oxygen concentration in the enzymatic reaction mixture. The present invention is based on these findings.

According to the present invention, the following inventions are provided:
(1) a method for producing fructooligosaccharide, wherein, in a reaction where fructooligosaccharide is produced by reacting β-fructofuranosidase with sucrose and a reaction step where glucose by-produced by said reaction is decreased by glucose oxidase, an amount of dissolved oxygen is adjusted to 2 to 8 ppm;
(2) a method for producing fructooligosaccharide, comprising the steps of: preparing a reaction mixture containing sucrose, β-fructofuranosidase, and glucose oxidase; and incubating the reaction mixture under optimum conditions for the β-fructofuranosidase, wherein, in the incubating step, an amount of dissolved oxygen is adjusted to 2 to 8 ppm;
(3) the method according to (1) or (2), wherein the fructooligosaccharide is a sugar solution having a content ratio of fructooligosaccharide of 90% or more;
(4) the method according to any of (1) to (3), which is carried out by using a reaction vessel equipped with an aeration means and a stirring means;
(5) the method according to (4), wherein the aeration means is a fine air bubble generating device;
(6) the method according to any one of (1) to (5), wherein the amount of dissolved oxygen is adjusted to 2 to 6 ppm, and preferably 3 to 6 ppm.

According to the present invention, a sugar solution having a content ratio of fructooligosaccharide of 90% or more can be obtained rapidly. Further, because the fructooligosaccharide content in the sugar solution obtained is high, fructooligosaccharide of high purity can be provided inexpensively without using an additional separation and purification means such as chromatographic separation and the like. Further, even though production efficiency usually drops relative to a laboratory level when a process is scaled up to an industrial production level, employment of a method of the present invention enables a sugar solution, having a content ratio of fructooligosaccharide of 90% or more, to be obtained even after a scale up. The fructooligosaccharide obtained by a method for producing fructooligosaccharide of the present invention can be used suitably for products such as various foods and drinks, medicines, and the like.

### DETAILED DESCRIPTION OF THE INVENTION

The present invention is a method for producing fructooligosaccharide, wherein, in a reaction where fructooligosaccharide is produced by reacting β-fructofuranosidase with sucrose and a reaction step where glucose by-produced by the reaction is decreased by glucose oxidase, an amount of dissolved oxygen is adjusted to 2 to 8 ppm.

Furthermore, the reaction process can be achieved by preparing a reaction mixture containing sucrose and necessary enzymes, and placing the same under optimum conditions. Therefore, according to another embodiment of the present invention, there is provided a method for producing fructooligosaccharide, comprising the steps of: preparing a reaction mixture containing sucrose, β-fructofuranosidase, and glucose oxidase; and incubating the reaction mixture under optimum conditions for the β-fructofuranosidase, wherein, in the incubating step, an amount of dissolved oxygen is adjusted to 2 ppm or more.

In the present invention, the term "fructooligosaccharide" means 1-kestose (GF2), nystose (GF3), fructosylnystose (GF4), and a mixture of these.

In the method of the present invention, as the β-fructofuranosidase, there can be used one having fructose transfer activity such that conversion from sucrose to 1-kestose becomes 40% or more.

Origin of the β-fructofuranosidase to be used is not particularly limited, but includes *Aspergillus, Penicilium,* or *Scopulariopsis.* Preferable is one derived from *Aspergillus niger* or *Aspergillus japonicus,* and more preferable is one derived from *Aspergillus japonicus* strain ATCC 20611.

In the method of the present invention, the reaction of sucrose and β-fructofuranosidase is carried out in a solution. A specific example is given as follows. A concentration of sucrose in a reaction mixture may be suitably selected considering specific activity of the enzyme, reaction temperature, and the like as long as sucrose is in a soluble range. For example, the concentration of sucrose is preferably in a range of 5 to 80% by weight, and more preferably in a range of 20 to 70% by weight.

An amount of β-fructofuranosidase to be added may be an amount sufficient to utilize sucrose in the enzymatic reaction mixture. For example, the amount is adjusted so that it becomes 1 U to 30 U, and preferably 2 U to 15 U per 1 g of sucrose. Here, the aforementioned enzyme unit (U) of β-fructofuranosidase is defined as follows. When 2.0 mL of a 25% sucrose solution, 1.0 mL of an enzyme solution, and 2.0 mL of a McIlvaine buffer solution (pH 5.0) are mutually mixed and reacted at 40°C for 60 minutes, an amount of the enzyme which produces 1 µmol of 1-kestose per 1 minute is defined as 1 U.

The reaction time of sucrose and β-fructofuranosidase until a content ratio of fructooligosaccharide becomes the maximum may be varied suitably. For example, a time from the start of the enzymatic reaction until the content ratio of fructooligosaccharide in the reaction mixture becomes 90% or more can be set at 6 to 80 hours, and preferably at 6 to 27 hours.

According to the method of the present invention, fructooligosaccharide is produced by reacting β-fructofuranosidase with sucrose and, at the same time, glucose by-produced is converted to gluconic acid by glucose oxidase to decrease glucose.

As the glucose oxidase used in the method of the present invention, there can be used, for example, glucose oxidase derived from *Aspergillus niger,* one derived from *Penicillium chrysogenum*, or the like. Among commercial glucose oxidase preparations, there are cases where the preparation has invertase activity as sub-activity. Among the enzyme preparations containing invertase, there are some which strongly exhibit activity of hydrolyzing fructooligosaccharide produced. Therefore, it is desirable that glucose oxidase used for the method of the present invention has only weak invertase activity of hydrolyzing fructooligosaccharide or does not contain invertase activity.

An amount of glucose oxidase to be added may be an amount sufficient for converting glucose which is by-produced. For example, the amount of glucose oxidase is adjusted so that it becomes 1 U or more, and preferably 4 U or more per 1 g of sucrose. The aforementioned enzyme unit (U) of glucose oxidase is defined as follows. Glucose oxidase is made to react on glucose as a substrate to generate hydrogen peroxide. In the presence of the generated hydrogen peroxide, 4-aminoantipyrine, and phenol, peroxidase is made to act to generate a quinoimine dye, which is quantified by measurement at a wavelength of 500 nm. An amount of the enzyme which is required to oxidize 1 µmol of glucose per 1 minute under a condition of pH 7.0 is defined as 1 U.

In a reaction of converting glucose to gluconic acid by glucose oxidase, hydrogen peroxide is by-produced. Because there are cases where an enzyme is inactivated by an oxidizing action of hydrogen peroxide, it is desirable, if necessary, to add catalase which decomposes hydrogen peroxide.

Therefore, the method of the present invention further includes, in the reaction step of glucose and glucose oxidase, a method of removing by-produced hydrogen peroxide by catalase. As the catalase to be used, preferable is one derived from *Aspergillus niger, Micrococcus lysodeikticus*, or the like. Furthermore, the aforementioned method includes not only a combined use of a glucose oxidase preparation and a catalase preparation, but also a selected use of a commercial glucose oxidase preparation having catalase activity as sub-activity.

An amount of catalase to be added may be such that it is sufficient to convert by-produced hydrogen peroxide to oxygen and water. For example, the amount of catalase is adjusted to 10 U or more, and preferably 100 U or more per 1g of sucrose.

In the reaction of sucrose and β-fructofuranosidase, and in the reaction step of by-produced glucose and glucose oxidase, the reaction temperature and pH are preferably set under optimum conditions for β-fructofuranosidase. For example, it is preferable to set the temperature under a condition of about 20 to 70°C and the pH under a condition of about 4 to 10, and more preferably in ranges of 25 to 40°C and pH 5 to 8, respectively.

When glucose is converted to gluconic acid, the pH of the reaction mixture decreases due to the gluconic acid. When the pH of the reaction mixture decreases, there occurs termination of the reaction to convert sucrose to fructooligosaccharide, acid hydrolysis of the produced fructooligosaccharide, or the like. Therefore, it is desirable to add a neutralizing agent accordingly to the reaction mixture to adjust the pH to near neutrality. As the neutralizing agent which can be used, there may be mentioned sodium carbonate, calcium carbonate, calcium hydroxide, sodium hydroxide, potassium hydroxide, ammonia water, and the like.

The amount of dissolved oxygen in the reaction mixture can be measured by using a method heretofore known in the art such as, for example, a chemical analysis method (a titration method), an electrochemical analysis method (a diaphragm electrode method), a photochemical analysis method (a fluorescence method), and the like. However, the amount of dissolved oxygen is preferably measured by the electrochemical analysis method (the diaphragm electrode method).

Because the reaction of glucose and glucose oxidase is an oxidation reaction, the reaction requires oxygen. Therefore, in order to increase the amount of dissolved oxygen in the reaction mixture, it is desirable to stir the reaction mixture at a high speed while aerating the same. In order to maintain the amount of dissolved oxygen in the reaction mixture, an air flow rate should be at least 3 L/min or more, preferably 10 L/min or more, and more preferably 20 L/min or more.

In the method of the present invention, it is preferable not only to maintain the air flow rate but also to install an aeration means and a stirring means, which enable fine air bubbles to be retained in the reaction mixture. The aeration means and the stirring means are not limited as long as they enable fine air bubbles to be retained in the reaction mixture. The fine air bubbles refer to, among air bubbles generated in the reaction mixture, those having an air bubble diameter of a centimeter size, a millimeter size, and a micrometer size. Especially by supplying air bubbles of a micrometer size, a residence time thereof in the reaction mixture increases. By making fine air bubbles be retained in the reaction mixture, a reaction time from the start of the enzymatic reaction to obtention of a sugar solution having a content ratio of fructooligosaccharide of 90% or more is shortened.

As the aeration means in an enzymatic reaction system of about 2 L in size, it is preferable to install a pipe (an aeration pipe) in a reaction vessel, which supplies air continuously. In an enzymatic reaction system of about 2 L in size, fine air bubbles can be retained in the enzymatic reaction mixture by, in addition to the aeration through the pipe, high-speed stirring at a stirring rotations of 600 rpm or more by a stirring means such as a stirring blade and the like. More preferably, there is installed a device which generates fine air bubbles, that is, a "fine air bubble generating device" at an end of the pipe. As specific examples of the device, there may be mentioned an air stone (Ibuki air stone #150), an aerator (OHR Aerator: OHR Laboratory Corporation), Aqua Blaster (registered trade mark: Aience Ltd.), a micro bubble generating device, and the like.

When an enzymatic reaction on a scale exceeding 10 L is carried out, stirring at a high speed of 600 rpm or more gives rise to fears that the stirring blade may be destroyed due to metal fatigue and that a trouble may arise due to an overload on a motor. Therefore, in a scaled-up enzymatic reaction system, it is desirable to set the number of stirring rotations at 300 rpm or less, and preferably at 200 rpm or less. Especially in a glycosyltransferase reaction in which a sugar solution of a high concentration is handled as in the present invention, resistance of the sugar solution to the stirring blade is large, so that a load due to high-speed stirring becomes larger than in a general oxidoreductase reaction. When the scale of the reaction is 50 L or more, the number of stirring rotations is set at 50 rpm or less, and preferably at 20 rpm or less. In production on an industrial scale, there is a restriction in the number of stirring rotations and, therefore, it is especially preferable to introduce a device which generates fine air bubbles.

The fructooligosaccharide obtained by the method of the present invention is preferably a sugar solution having a content ratio of fructooligosaccharide of 90% or more. The term "content ratio of fructooligosaccharide" means a total content of 1-kestose, nystose, and fructosylnystose relative to a total content of neutral sugars (fructose, glucose, sucrose, 1-kestose, nystose, and fructosylnystose) contained in the sugar solution. A sugar composition of the neutral sugars can be analyzed, for example, under the following HPLC conditions: column, RT 250-4.0 LiChrosphor 100 NH2 (Cica-Reagent Co.); mobile phase, 66% acetonitrile; flow rate, 1 ml/min; column temperature, 40°C; detector, differential refractometer.

A heretofore known separation and purification treatment can be additionally performed on the sugar solution containing fructooligosaccharide. As the separation and purification treatment, there may be mentioned, for example, a treatment to adjust the gluconic acid content by a means such as electrodialysis and the like, a treatment to concentrate the sugar solution by a means such as an evaporator and the like, a treatment to dry the sugar solution by a means such as a vacuum dryer and the like.

The fructooligosaccharide obtained by the method of the present invention can be produced inexpensively and, from that point of view, can be widely applied to heretofore known drinks, foods, and medicines.

### EXAMPLES

The present invention will be described in detail with reference to the following Examples, but the present invention is by no means limited thereto.

### Reference Example 1: Preparation of β-Fructofuranosidase

A culture medium of 350 mL containing 2.0% of powdered bouillon, 5.0% of sucrose, and 0.5% of CMC was added to an Erlenmeyer flask and sterilized. Thereafter, *Aspergillus japonicus* strain ATCC 20611 was inoculated and cultured at 28°C for 20 hours to prepare a seed culture solution. A culture medium of 15 L containing 5.0% of sucrose, 3.6% of a yeast extract, and 0.5% of CMC was added to a 30 L jar fermenter, its pH was adjusted to 6.5, and thereafter the culture medium was sterilized at 120°C for 30 minutes. Subsequently, 350 mL of the seed culture solution was inoculated aseptically into the culture medium and cultured at 28°C for 72 hours. After culturing was complete, a culture solution was centrifuged and further freeze-dried to thereby obtain cells containing crude enzyme which had fructose transfer activity. The fructose transfer activity of the cells containing crude enzyme was 1,580 U/g (cell weight). In addition, the fructose transfer activity is a value obtained by defining an amount of enzyme as 1 U (unit), which is enough to produce 1 µmol of 1-kestose per 1 minute when 2.0 mL of a 25% sucrose solution, 1.0 mL of an enzyme solution, and 2.0 mL of a McIlvaine buffer solution (pH 5.0) are mutually mixed and reacted at 40°C for 60 minutes. The cells containing crude enzyme was freeze-dried and used as β-fructofuranosidase in subsequent tests.

### Example 1: Production of Fructooligosaccharide (1) 2 L Scale-1

An enzymatic reaction was carried out under the following conditions:
Substrate solution: 578 g of sucrose dissolved in 1350 g of distilled water (sucrose 30% by weight)
Enzyme solution: 5.19 U added per 1 g of sucrose
β-fructofuranosidase (Reference Example 1): 3,000 U
Glucose oxidase (product name: Hydelase 15, produced by Amano Enzyme Inc. and containing catalase activity as sub-activity): 3,000 U
Temperature: 30°C
pH: 7.0
Number of stirring rotations: 600 rpm
Air flow rate: 3 L/min (carried out continuously through a pipe having a bore diameter of 1 mm, the pipe being disposed on the bottom of a reaction vessel)

A reaction mixture was sampled from the start of an enzymatic reaction to 0 to 24 hours thereafter. The sampled reaction mixture was heated in a hot water bath set at 82°C for 30 minutes to inactivate the enzyme and thereby to terminate the reaction. Further, after adding activated carbon to the reaction mixture in a proportion of 0.3% relative to the weight of the substrate, the solution was filtered through a filter paper and a 0.45 µm filter.

Each reaction mixture obtained was analyzed for sugar composition. The sugar composition analysis was carried out under the following conditions: column, RT 250-4.0 LiChrosphor 100 NH2 (Cica-Reagent Co.); mobile phase, 66% acetonitrile; flow rate, 1 ml/min; column temperature, 40°C; detector, differential refractometer. Relative to a total content of 100% of neutral sugars including fructose, glucose, sucrose, 1-kestose, nystose, and fructosylnystose, a content ratio of each component was obtained from the content of each. The content ratio of fructooligosaccharide was calculated by summing content ratios of 1-kestose, nystose, and fructosylnystose. The results are shown in Table 1. Abbreviations in the table mean the following:
F: fructose
G: glucose
GF: sucrose
GF2: 1-kestose
GF3: nystose
GF4: fructosylnystose
FOS: fructooligosaccharide (sum of GF2 + GF3 + GF4)

**[Table 1]**

| Content ratio (%) | Reaction time (hr) | | | | | | | | |
|---|---|---|---|---|---|---|---|---|---|
| | 0 | 2 | 4 | 20 | 21 | 22 | 23 | 24 | A. I. ^{*1} |
| F | 0.0 | 0,0 | 0.7 | 2.1 | 2.3 | 2.4 | 2.8 | 2.6 | 3.0 |
| G | 0.0 | 3.1 | 4.0 | 0.9 | 0.5 | 0.0 | 0.0 | 0.0 | 0.5 |
| GF | 100.0 | 75.7 | 57.5 | 2.7 | 2.1 | 3.0 | 2.8 | 2.3 | 1.5 |
| GF2 | 0.0 | 20.2 | 35.0 | 38.2 | 35.7 | 32.0 | 28.4 | 26.8 | 25.1 |
| GF3 | 0.0 | 1.0 | 2.8 | 52.0 | 55.2 | 57.2 | 59.4 | 60.9 | 61.8 |
| GF4 | 0.0 | 0.0 | 0.0 | 4.1 | 4.2 | 5.4 | 6.6 | 7.4 | 8.1 |
| FOS(%) | 0.0 | 21.2 | 37.8 | 94.3 | 95.1 | 94.6 | 94.4 | 95.1 | 95.0 |

| | | | | | | | | | |
|---|---|---|---|---|---|---|---|---|---|
| *1: After inactivation | | | | | | | | | |

From the results of Table 1, a content ratio of fructooligosaccharide became 90% or more after 20 hours from the start of the enzymatic reaction. After 24 hours from the start of the enzymatic reaction, a sugar solution having a content ratio of fructooligosaccharide of 95% or more was obtained. When an amount of dissolved oxygen of the solution during the enzymatic reaction was measured by using a portable dissolved oxygen meter (DKK-TOA Corporation: DO-14P), the amount of dissolved oxygen was 3 ppm or more (3 to 6 ppm) during a reaction time from 2 to 24 hours.

### Example 2: Production of Fructooligosaccharide (2) 2 L Scale-2

An enzymatic reaction was carried out under the following conditions:
Substrate solution: 578 g of sucrose dissolved in 1350 g of distilled water (sucrose 30% by weight)
Enzyme solution: 5.19 U added per 1 g of sucrose
β-fructofuranosidase (Reference Example 1): 3,000 U
Glucose oxidase (product name: Hydelase 15, see Example 1): 3,000 U
Temperature: 30°C
pH: 7.0
Number of stirring rotations: A) 400 rpm, B) 600 rpm, C) 800 rpm
Air flow rate: 3 L/min (carried out continuously through a pipe having a bore diameter of 1 mm, the pipe being disposed on the bottom of a reaction vessel)

An amount of dissolved oxygen in the solution during the enzymatic reaction was measured by using a portable dissolved oxygen meter (DKK-TOA Corporation: DO-14P). A reaction mixture was sampled from the start of the enzymatic reaction to 0 to 30 hours (35 hours for a 400 rpm Test Section) thereafter. The sampled reaction mixture was treated in the same manner as in Example 1 and was analyzed for the sugar composition.

Results for Test Sections where the number of stirring rotations was 400 rpm, 600 rpm, and 800 rpm are shown in Table 2, Table 3, and Table 4, respectively. Among abbreviations in the Table, "DO" means "dissolved oxygen concentration."

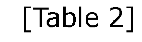

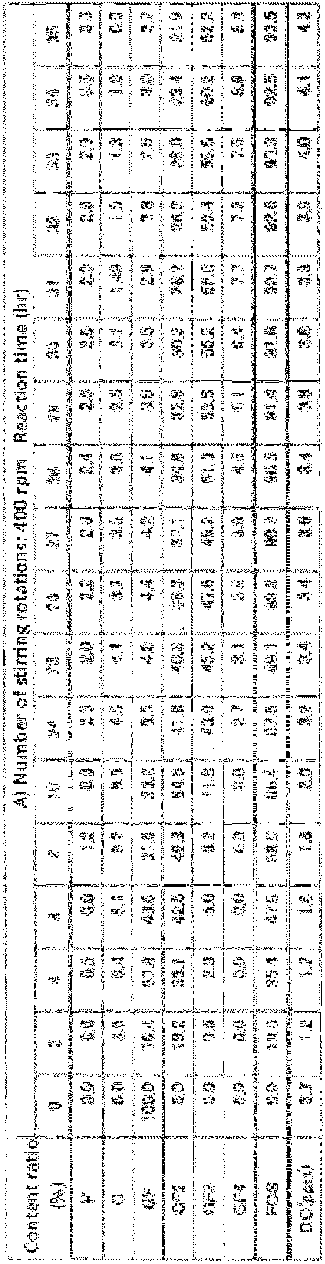

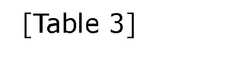

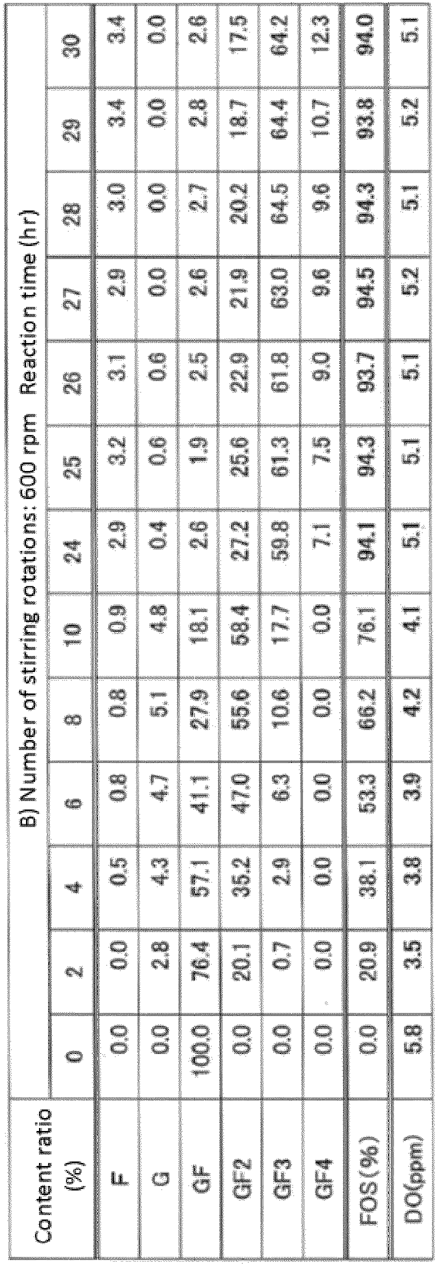

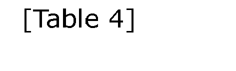

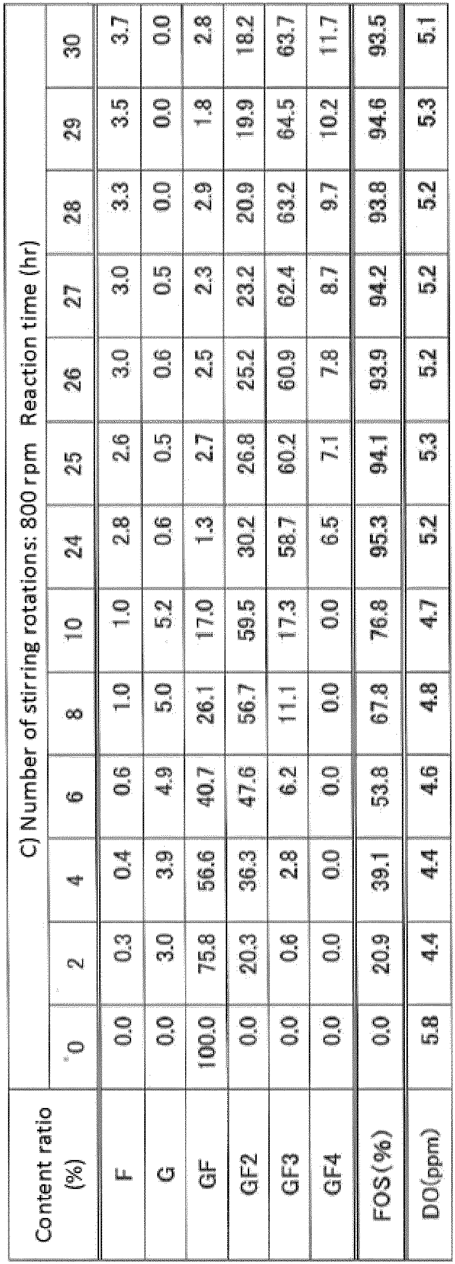

From Table 2, when the number of stirring rotations was 400 rpm, the amount of dissolved oxygen from the start of the reaction until 2 to 8 hours has passed was less than 2 ppm. Further, after 24 hours of reaction time, the amount of dissolved oxygen was maintained at 3 ppm or more (3.2 to 4.2 ppm). When the number of stirring rotations was 400 rpm, a content ratio of fructooligosaccharide became 90% or more after 27 hours from the start of the reaction.

From Table 3 and Table 4, when the number of stirring rotations was 600 to 800 rpm, the amount of dissolved oxygen was maintained at 3 ppm or more (3.5 to 5.8 ppm) from the start of the reaction until 30 hours had passed. When 24 hours had passed from the start of the reaction, the content ratio of fructooligosaccharide in the reaction mixture became 90% or more.

From the results of Tables 2 to 4, a sugar solution having a content ratio of fructooligosaccharide of 90% or more could be obtained, in a scale of 2 L, by adjusting the amount of dissolved oxygen in the enzymatic reaction mixture to at least 2 ppm or more (2 to 6 ppm), and preferably to 3 ppm or more (3 to 6 ppm).

In a scale of 2 L, air supplied from the pipe having a bore diameter of 1 mm, the pipe being disposed on the bottom of the reaction vessel, was being finely dispersed in the solution by a rotating stirring blade. Especially when the number of stirring rotations was 600 rpm or more, air bubbles were well distributed in the reaction vessel in a very fine state. Therefore, it is thought that, when the number of stirring rotations was 600 rpm or more, oxygen necessary for the glucose oxidase reaction was supplied in a sufficient amount at any period of time during the enzymatic reaction.

### Example 3: Production of Fructooligosaccharide (3) 2 L Scale-3

An enzymatic reaction was carried out under the following conditions. In Example 3, the amount of enzyme added was 2 to 3 times that in Example 1 and Example 2:
Substrate solution: 578 g of sucrose dissolved in 1350 g of distilled water (sucrose 30% by weight)
Enzyme solution:
   β-Fructofuranosidase (Reference Example 1): 8,764 U (15.2 U added per 1 g of sucrose)
Glucose oxidase (product name: Hydelase 15, see Example 1): 9,000 U (15.6 U added per 1 g of sucrose)
Temperature: 30°C
pH: 7.0
Number of stirring rotations: A) 300 rpm, B) 400 rpm, C) 500 rpm, D) 600 rpm, E) 700 rpm, F) 800 rpm
Air flow rate: 3 L/min (carried out continuously through a pipe having a bore diameter of 1 mm, the pipe being disposed on the bottom of a reaction vessel)

An amount of dissolved oxygen in the solution during the enzymatic reaction was measured by using a portable dissolved oxygen meter (DKK-TOA Corporation: DO-14P). A reaction mixture was sampled from the start of an enzymatic reaction to 0 to 8 hours thereafter. The sampled reaction mixture was treated in the same manner as in Example 1 and was analyzed for the sugar composition. The results are shown in Tables 5 to 10.

**[Table 5]**

| Content ratio (%) | A) Number of stirring rotations: 300 rpm Reaction time (hr) | | | | | | | | |
|---|---|---|---|---|---|---|---|---|---|
| | 0 | 1 | 2 | 3 | 4 | 5 | 6 | 7 | 8 |
| F | 0,0 | 0.5 | 0.7 | 0.9 | 1.1 | 1.2 | 1.4 | 1.6 | 1.7 |
| G | 0.0 | 7.8 | 12.1 | 15.2 | 16.6 | 18.0 | 18.7 | 19.0 | 18.9 |
| GF | 100.0 | 62.9 | 44.6 | 29.5 | 22.2 | 16.8 | 13.7 | 12.1 | 11.0 |
| GF2 | 0.0 | 27.2 | 38.7 | 46.8 | 49.3 | 49.5 | 49.6 | 47.5 | 46.0 |
| GF3 | 0.0 | 1.6 | 3.9 | 7.6 | 10.9 | 14.5 | 16.6 | 19.9 | 22.3 |
| GF4 | 0.0 | 0.0 | 0.0 | 0.0 | 0.0 | 0.0 | 0.0 | 0.0 | 0.0 |
| FOS(%) | 0.0 | 28.8 | 42.6 | 54.3 | 60.2 | 64.0 | 66.2 | 67.3 | 68.3 |
| DO(ppm) | 5.4 | 0.3 | 0.3 | 0.3 | 0.3 | 0,4 | 0.4 | 0.3 | 0.3 |

**[Table 6]**

| Content ratio (%) | B) Number of stirring rotations: 400 rpm Reaction time (hr) | | | | | | | | |
|---|---|---|---|---|---|---|---|---|---|
| | 0 | 1 | 2 | 3 | 4 | 5 | 6 | 7 | 8 |
| F | 0.0 | 0.4 | 0.5 | 0.8 | 1.0 | 1.1 | 1.4 | 1.6 | 1.8 |
| G | 0.0 | 7.8 | 11.3 | 15.7 | 13.4 | 16.5 | 16.7 | 16.8 | 16.3 |
| GF | 100.0 | 62.8 | 44.0 | 22.0 | 27.2 | 16.6 | 13.2 | 11.2 | 10.0 |
| GF2 | 0.0 | 27.5 | 39.9 | 50.3 | 44.8 | 51.0 | 50.5 | 49.0 | 46.8 |
| GF3 | 0.0 | 1.5 | 4.2 | 11.2 | 13.6 | 14.9 | 18.3 | 21.4 | 24.0 |
| GF4 | 0.0 | 0.0 | 0.0 | 0.0 | 0.0 | 0.0 | 0.0 | 0.0 | 1.0 |
| FOS(%) | 0.0 | 29.0 | 44.1 | 61.5 | 58.4 | 65.8 | 68.8 | 70.4 | 71.8 |
| DO(ppm) | 5.4 | 0.5 | 0.6 | 0.5 | 0.6 | 0.5 | 0.6 | 0.6 | 0.6 |

**[Table 7]**

| Content ratio (%) | C) Number of stirring rotations: 500 rpm Reaction time (hr) | | | | | | | | |
|---|---|---|---|---|---|---|---|---|---|
| | 0 | 1 | 2 | 3 | 4 | 5 | 6 | 7 | 8 |
| F | 0.0 | 0.3 | 0.6 | 0.9 | 0.9 | 1.1 | 1.4 | 1.6 | 1.7 |
| G | 0.0 | 7.0 | 10.1 | 12.5 | 12.9 | 13.0 | 12.2 | 16.7 | 10.1 |
| GF | 100.0 | 63.9 | 44.3 | 27.2 | 20.8 | 15.1 | 11.8 | 11.3 | 8.4 |
| GF2 | 0.0 | 27.2 | 40.7 | 50.2 | 52.7 | 53.6 | 52.5 | 49.0 | 47.9 |
| GF3 | 0.0 | 1.5 | 4.4 | 9.2 | 12.6 | 17.3 | 22.0 | 21.5 | 30.6 |
| GF4 | 0.0 | 0.0 | 0.0 | 0.0 | 0.0 | 0.0 | 0.0 | 0.0 | 1.3 |
| FOS(%) | 0,0 | 28.8 | 45.0 | 59.4 | 65.4 | 70.9 | 74.5 | 70.5 | 79.7 |
| DO(ppm) | 5.5 | 0.8 | 0.9 | 1.0 | 1.1 | 1.2 | 1.4 | 1.4 | 1.4 |

**[Table 8]**

| Content ratio (%) | D) Number of stirring rotations: 600 rpm Reaction time (hr) | | | | | | | | |
|---|---|---|---|---|---|---|---|---|---|
| | 0 | 1 | 2 | 3 | 4 | 5 | 6 | 7 | 8 |
| F | 0.0 | 0.5 | 0.6 | 1.2 | 1.3 | 1.6 | 1.8 | 1.8 | 2.2 |
| G | 0.0 | 6.6 | 8.7 | 10.5 | 10.1 | 8.2 | 6.2 | 4.3 | 1.9 |
| GF | 100.0 | 62.4 | 42.4 | 22.7 | 14.5 | 9.7 | 7.4 | 5.2 | 3.7 |
| GF2 | 0.0 | 28.8 | 43.3 | 53.4 | 55.6 | 54.0 | 50.7 | 46.4 | 41.1 |
| GF3 | 0.0 | 1.6 | 5.0 | 12.3 | 18.5 | 26.4 | 32.6 | 40.0 | 47.9 |
| GF4 | 0.0 | 0.0 | 0.0 | 0.0 | 0.0 | 0.0 | 1.3 | 2.3 | 3.2 |
| FOS(%) | 0.0 | 30.5 | 48.3 | 65.7 | 74.1 | 80.4 | 84.6 | 88.7 | 92.2 |
| DO(ppm) | 5.5 | 1.5 | 1.5 | 1.8 | 2.2 | 2.6 | 2.7 | 3.0 | 3.7 |

**[Table 9]**

| Content ratio (%) | E) Number of stirring rotations: 700 rpm Reaction time (hr) | | | | | | | | | |
|---|---|---|---|---|---|---|---|---|---|---|
| | 0 | 1 | 2 | 3 | 4 | 5 | 6 | 7 | 8 | 9 |
| F | 0.0 | 0.0 | 0.6 | 0.8 | 1.2 | 1.4 | 1.7 | 2.1 | 2.3 | 2.8 |
| G | 0.0 | 6.1 | 7.6 | 8.4 | 7.6 | 6.1 | 3.8 | 1.9 | 1.0 | 0.7 |
| GF | 100.0 | 62.6 | 42.4 | 24.8 | 15.0 | 9.1 | 5.8 | 4.6 | 3.7 | 2.8 |
| GF2 | 0.0 | 29.2 | 44.4 | 54.6 | 57.4 | 56.2 | 51.2 | 44.7 | 30.9 | 29.6 |
| GF3 | 0.0 | 2.1 | 5.0 | 11.3 | 18.8 | 26.2 | 35.9 | 44.2 | 51.9 | 58.0 |
| GF4 | 0.0 | 0.0 | 0.0 | 0.0 | 0.0 | 0.9 | 1.5 | 2.5 | 4.2 | 6.1 |
| FOS(%) | 0.0 | 31.2 | 49.4 | 66.0 | 76.2 | 83.3 | 88.7 | 91.4 | 93.0 | 93.7 |
| DO(ppm) | 5.5 | 2.3 | 2.6 | 2.6 | 3.1 | 3.6 | 3.9 | 4.2 | 4.7 | 4.9 |

**[Table 10]**

| Content ratio (%) | F) Number of stirring rotations: 800 rpm Reaction time (hr) | | | | | | | | |
|---|---|---|---|---|---|---|---|---|---|
| | 0 | 1 | 2 | 3 | 4 | 5 | 6 | 7 | 8 |
| F | 0.0 | 0.5 | 0.7 | 0.9 | 1.3 | 1.6 | 2.1 | 2.4 | 2.5 |
| G | 0.0 | 6.0 | 7.5 | 7.0 | 5.3 | 3.1 | 1.6 | 1.1 | 0.7 |
| GF | 100.0 | 60.4 | 38.3 | 20.7 | 12.3 | 6.6 | 4.2 | 2.3 | 1.9 |
| GF2 | 0.0 | 30.9 | 46.5 | 57.1 | 58.8 | 55.3 | 48.2 | 39.4 | 32.2 |
| GF3 | 0.0 | 2.2 | 7.1 | 14.3 | 22.4 | 31.7 | 41.8 | 51.0 | 57.1 |
| GF4 | 0.0 | 0.0 | 0.0 | 0.0 | 0.0 | 1.6 | 2.0 | 4.0 | 5.6 |
| FOS(%) | 0.0 | 33.1 | 53.6 | 71.4 | 81.1 | 88.7 | 92.0 | 94.3 | 94.8 |
| DO(ppm) | 5.6 | 2.4 | 2.7 | 3.1 | 3.5 | 3.8 | 4.4 | 4.7 | 4.9 |

When the number of stirring rotations was 500 rpm or less (300 rpm (Table 5), 400 rpm (Table 6), and 500 rpm (Table 7)), a sugar solution having a content ratio of fructooligosaccharide of 90% or more could not be obtained because, in the present Example, the enzymatic reaction time was limited to 8 hours. Further, in every Test Section of Tables 5 to 7, the amount of dissolved oxygen was less than 2 ppm.

When the number of stirring rotations was 600 rpm or more (600 rpm (Table 8), 700 rpm (Table 9), and 800 rpm (Table 10)), a sugar solution having a content ratio of fructooligosaccharide of 90% or more could be obtained within 8 hours from the start of the enzymatic reaction. When the number of stirring rotations was 700 rpm or more (Tables 9 to 10), the amount of dissolved oxygen was 2 ppm or more (2.3 to 5.6 ppm) at all periods of time during the enzymatic reaction. When the stirring speed was 600 rpm (Table 8), the amount of dissolved oxygen was 2 ppm or more (2.2 to 3.7 ppm) after 4 hours from the start of the enzymatic reaction.

From the results of Tables 5 to 10, a sugar solution having a content ratio of fructooligosaccharide of 90% or more could be obtained, in a scale of 2 L, by adjusting the amount of dissolved oxygen in the enzymatic reaction mixture to at least 2 ppm or more (2 to 6 ppm), and preferably to 3 ppm or more (3 to 6 ppm). Furthermore, the conditions of aeration and stirring in order to maintain the aforementioned amount of dissolved oxygen were 3 L/min or more in terms of the air flow rate and 600 rpm or more (600 to 800 rpm) in terms of the number of stirring rotations.

### Example 4: Production of Fructooligosaccharide (4) 10 L Scale

An enzymatic reaction was carried out under the following conditions:
Substrate solution: 2890 g of sucrose dissolved in 6750 g of distilled water (sucrose 30% by weight)
Enzyme solution:
   β-fructofuranosidase (Reference Example 1): 15,000 U (5.19 U added per 1 g of sucrose)
Glucose oxidase (product name: Hydelase 15, see Example 1): 13,000 U (4.50 U added per 1 g of sucrose)
Temperature: 30°C
pH: 7.0
Number of stirring rotations: 150 rpm
Air flow rate:
   A) 3 L/min (carried out continuously through Ibuki air stone 30 mm×150 mm #150 (average pore diameter 12 µm, maximum 44 µm));
   B) 10 L/min (carried out continuously through Ibuki air stone 30 mm×150 mm #150 (average pore diameter 12 µm, maximum 44 µm))

An amount of dissolved oxygen in the solution during the enzymatic reaction was measured by using a portable dissolved oxygen meter (DKK-TOA Corporation: DO-14P). A reaction mixture was sampled from the start of the enzymatic reaction to 0 to 5 hours thereafter for Test Section A, and from the start of the enzymatic reaction to 0 to 22 hours thereafter for Test Section B. The sampled reaction mixture was treated in the same manner as in Example 1 and was analyzed for the sugar composition. The results are shown in Tables 11 to 12.

**[Table 11]**

| Content ratio (%) | A) Air flow rate: 3 L/min Reaction time (hr) | | | | | | | |
|---|---|---|---|---|---|---|---|---|
| | 0 | 0.5 | 1 | 1.5 | 2 | 3 | 4 | 5 |
| F | 0.0 | 0.0 | 0.0 | 0.0 | 0.0 | 0.5 | 0.5 | 0.6 |
| G | 0.0 | 2.5 | 3.9 | 5.0 | 6.1 | 8.4 | 9.9 | 10.8 |
| GF | 100.0 | 88.5 | 82.5 | 76.4 | 70.6 | 59.1 | 48.6 | 43.5 |
| GF2 | 0.0 | 9.0 | 13.2 | 17.9 | 22.0 | 29.9 | 37.6 | 40.5 |
| GF3 | 0.0 | 0.0 | 0.4 | 0.7 | 1.3 | 2.1 | 3.4 | 4.6 |
| GF4 | 0.0 | 0.0 | 0.0 | 0.0 | 0.0 | 0.0 | 0.0 | 0.0 |
| FOS(%) | 0.0 | 9.0 | 13.6 | 18.6 | 23.3 | 32.0 | 41.0 | 45.1 |
| DO(ppm) | 4.1 | 2.8 | 1.8 | 1.5 | 1.2 | 1.2 | 1.2 | 1.1 |

**[Table 12]**

| Content ratio (%) | B) Air flow rate: 10 L/min Reaction time (hr) | | | | | | | | | | | | |
|---|---|---|---|---|---|---|---|---|---|---|---|---|---|
| | 0 | 1 | 2 | 3 | 4 | 15 | 16 | 17 | 18 | 19 | 20 | 21 | 22 |
| F | 0.0 | 0.4 | 0.3 | 0.4 | 0.5 | 2.0 | 2.1 | 2.4 | 2.2 | 2.5 | 2.8 | 3.3 | 2.8 |
| G | 0.0 | 2.6 | 4.2 | 4.7 | 5.4 | 1.9 | 1.5 | 1.0 | 0.6 | 0.5 | 0.0 | 0.4 | 0.0 |
| GF | 100.0 | 84.3 | 68.7 | 60.1 | 51.4 | 3.4 | 3.4 | 2.8 | 2.5 | 2.3 | 2.8 | 2.6 | 2.7 |
| GF2 | 0.0 | 12.7 | 25.4 | 32.5 | 39.3 | 41.0 | 36.7 | 33.2 | 30.6 | 26.5 | 24.6 | 22.4 | 21.1 |
| GF3 | 0.0 | 0.0 | 1.4 | 2.3 | 3.4 | 46.9 | 50.8 | 53.6 | 56.4 | 59.2 | 60.1 | 60.3 | 61.4 |
| GF4 | 0.0 | 0.0 | 0.0 | 0.0 | 0.0 | 4.8 | 5.5 | 7.0 | 7.7 | 9.0 | 9.7 | 11.0 | 12.0 |
| FOS(%) | 0.0 | 12.7 | 26.8 | 34.8 | 42.7 | 92.7 | 93.0 | 93.8 | 94.7 | 94.7 | 94.4 | 93.7 | 94.5 |
| DO(ppm) | 5.8 | 3.6 | 3.4 | 3.5 | 3.3 | 2.9 | 2.9 | 3.2 | 3.3 | 3.4 | 3.8 | 3.9 | 3.8 |

In a scale of 10 L which is about 5 times that in Examples 1 to 3, it is difficult to stir at a high speed of 600 rpm or more from the viewpoint of safety and, therefore, the number of stirring rotations was set at 150 rpm. In the present Example, an air stone was introduced as an aeration means, whereupon fine air bubbles were supplied continuously in the enzymatic reaction mixture. In Test Section A, the enzymatic reaction was started at an air flow rate of 3 L/min. After 1 hour from the start of the reaction, the amount of dissolved oxygen became less than 2 ppm, and thereafter the amount of dissolved oxygen showed a tendency to decrease. On the other hand, a content ratio of glucose continued to increase after the enzymatic reaction started and exceeded 10% after 5 hours from the start of the reaction. In this Test Section, a sugar solution having a content ratio of fructooligosaccharide of 90% or more could not be obtained. The reason why the content ratio of glucose increased was thought to be that the glucose oxidase reaction did not proceed smoothly.

In Test Section B, the enzymatic reaction was started with the air flow rate set at 10 L/min. The amount of dissolved oxygen was maintained at about 3 to 6 ppm (2.9 to 5.8 ppm) for a period from the start of the reaction to 22 hours thereafter. In Test Section B, a sugar solution having a content ratio of fructooligosaccharide of 90% or more could be obtained after 15 hours from the start of the enzymatic reaction.

From the results of Tables 11 to 12, a sugar solution having a content ratio of fructooligosaccharide of 90% or more could be obtained, in a scale of 10 L, by adjusting the amount of dissolved oxygen in the enzymatic reaction mixture to at least 2 ppm or more (2 to 6 ppm), and preferably to 3 ppm or more (3 to 6 ppm). Furthermore, the conditions of aeration and stirring in order to maintain the aforementioned amount of dissolved oxygen were 10 L/min or more in terms of the air flow rate and 150 rpm or more in terms of the number of stirring rotations. Because it is difficult to stir at a high speed at 600 rpm or more in a scale of 10 L, it was useful to introduce, as an aeration means, a device for generating fine air bubbles.

### Example 5: Production of Fructooligosaccharide (5) 50 L Scale

An enzymatic reaction was carried out under the following conditions:
Substrate solution: 17.34 kg of sucrose dissolved in 40.5 kg of distilled water (sucrose 30% by weight)
Enzyme solution:
   β-fructofuranosidase (Reference Example 1): 89,880 U (5180 U added per 1 kg of sucrose)
Glucose oxidase (product name: Hydelase 15, see Example 1): 89,700 U (5170 U added per 1 kg of sucrose)
Temperature: 30°C
pH: 7.0
Number of stirring rotations: 100 rpm
Air flow rate: 20 L/min (4 units of Ibuki air stone 30 mm×150 mm #150 (average pore diameter 12 µm, maximum 44 µm) were used, each with an air flow rate of 5 L/min)

An amount of dissolved oxygen in the solution during the enzymatic reaction was measured by using a portable dissolved oxygen meter (DKK-TOA Corporation: DO-14P). A reaction mixture was sampled from the start of the enzymatic reaction to 0 to 28 hours thereafter. The sampled reaction mixture was treated in the same manner as in Example 1 and was analyzed for the sugar composition. The results are shown in Table 13.

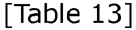

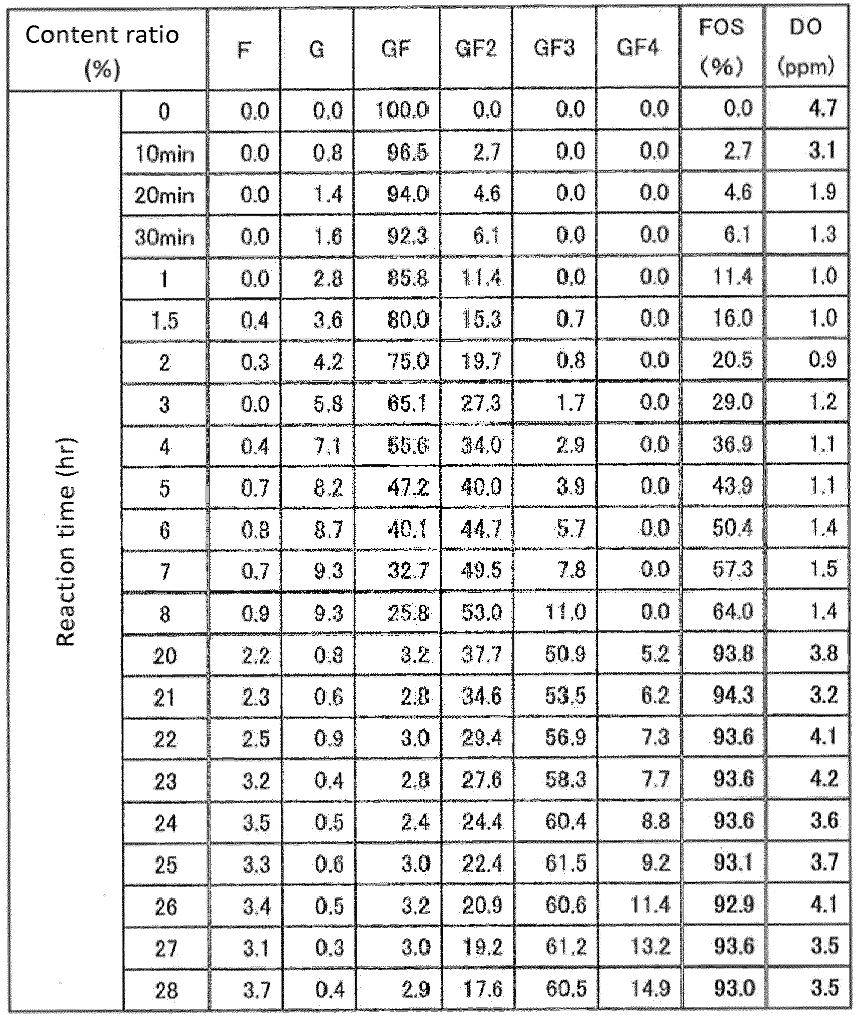

From Table 13, the amount of dissolved oxygen decreased from immediately after the start of the enzymatic reaction and dropped to 1 ppm after 1 hour. From after 6 hours from the start of the reaction, a tendency was seen that the amount of dissolved oxygen increased. At the same time, a tendency was seen that the content ratio of fructooligosaccharide increased. After 20 hours from the start of the reaction, a sugar solution having a content ratio of fructooligosaccharide of 90% or more could be obtained.

From the results of Table 13, a sugar solution having a content ratio of fructooligosaccharide of 90% or more could be obtained, in a scale of 50 L, by adjusting the amount of dissolved oxygen in the enzymatic reaction mixture to at least 2 ppm or more, and preferably to 3 ppm or more (3 to 5 ppm). Furthermore, the conditions of aeration and stirring in order to maintain the aforementioned amount of dissolved oxygen were 20L/min or more in terms of the air flow rate and 100 rpm or more in terms of the stirring rotations. Because it is difficult to stir at a high speed of 600 rpm or more in a scale of 50 L, it was useful to introduce, as an aeration means, a device for generating fine air bubbles.

### Comparative Example 1: Production of Fructooligosaccharide 4 T Scale

An enzymatic reaction was carried out under the following conditions:
Substrate solution: 4000 kg of sucrose dissolved in 9300 kg of distilled water (sucrose 30% by weight)
Enzyme solution:
   β-fructofuranosidase (Reference Example 1): 5200 U added per 1 kg of sucrose
Glucose oxidase (product name: Hydelase 15, see Example 1): 6300 U added per 1 kg of sucrose
Temperature: 30°C
pH: 6 to 7
Number of stirring rotations: 20 rpm
Air flow rate: 15.6 L/min (carried out continuously through a mesh disposed in a pipe having a 4 cm diameter at about 20 cm from an end, the pipe being disposed on the bottom of a reaction vessel and the mesh having fine holes with a hole diameter of 25 µm)

An amount of dissolved oxygen in the solution during the enzymatic reaction was measured by using a portable dissolved oxygen meter (DKK-TOA Corporation: DO-14P). A reaction mixture was sampled after 0 hour, 24 hours, and 80 hours from the start of the enzymatic reaction. The sampled reaction mixture was treated in the same manner as in Example 1 and was analyzed for the sugar composition.

The sugar composition of a solution at the start of the enzymatic reaction had a sucrose content ratio of 100%. The sugar composition after 24 hours from the start of the reaction was as follows:
fructose: 1.2%
glucose: 25.5%
sucrose: 11.4%
GF2: 39.7%
GF3: 21.2%
GF4: 1.0%
fructooligosaccharide (GF2+GF3+GF4): 61.9%

In a reaction system of 4 t scale, the amount of dissolved oxygen in the enzymatic reaction mixture was less than 2 ppm. The reaction mixture after 24 hours from the start of the reaction had a content ratio of fructooligosaccharide of 61.9%. Further, when a sugar composition of the reaction mixture after 80 hours was analyzed, the content ratio of fructooligosaccharide was 70%. In this Test Section, a sugar solution having a content ratio of fructooligosaccharide of 90% or more could not be obtained.

### Example 6: Production of Fructooligosaccharide (6) 4 T Scale

An enzymatic reaction was carried out under the following conditions:
Substrate solution: 4000 kg of sucrose dissolved in 9300 kg of distilled water (sucrose 30% by weight)
Enzyme solution:
   β-fructofuranosidase (Reference Example 1): 5200 U added per 1 kg of sucrose
Glucose oxidase (product name: Hydelase 15, see Example 1): 6300 U added per 1 kg of sucrose
Temperature: 30°C
pH: 6 to 7
Number of stirring rotations: 20 rpm
Air flow rate: 15.6 L/min

Aeration was carried out continuously through a "pipe a" having a diameter of 4 cm, the pipe being disposed on the bottom of a reaction vessel. A "pipe b" having a diameter of 15 cm was disposed in such a way as to cover the "pipe a". An end of the "pipe b" was disposed so that it protruded from the end of the "pipe a" by about 10 cm. On the endmost part of the "pipe b", which was made to protrude from the "pipe a", there was arranged a plate portion equipped with a twist. When aeration was carried out by the aeration means, fine air bubbles were generated in the reaction mixture by occurrence of underflow between the "pipe a" and the "pipe b" involving the reaction mixture, and the fine air bubbles were retained in the reaction mixture.

An amount of dissolved oxygen in the solution during the enzymatic reaction was measured by using a portable dissolved oxygen meter (DKK-TOA Corporation: DO-14P). A reaction mixture was sampled after 0 hour and 80 hours from the start of the enzymatic reaction. The sampled reaction mixture was treated in the same manner as in Example 1 and was analyzed for the sugar composition.

The amount of dissolved oxygen in the enzymatic reaction was 2 ppm. The sugar composition at the start of the enzymatic reaction had a content ratio of sucrose of 100 %. The sugar composition after 80 hours from the start of the reaction was as follows:
fructose: 4.0%
glucose: 0%
sucrose: 5.6%
GF2: 23.0%
GF3: 55.9%
GF4: 11.5%
fructooligosaccharide (GF2+GF3+GF4): 90.4%

A sugar solution having a content ratio of fructooligosaccharide of 90% or more could be obtained, in a scale of 50 L, by adjusting the amount of dissolved oxygen in the enzymatic reaction mixture to at least 2 ppm or more. Furthermore, the conditions of aeration and stirring in order to maintain the aforementioned amount of dissolved oxygen were 15 L/min or more in terms of the air flow rate and 20 rpm or more in terms of the number of stirring rotations. Even though the air flow rate was the same in the present Example and in the Comparative Example 1, a sugar solution having a content ratio of fructooligosaccharide of 90% or more could be obtained by introducing, as an aeration means, a device which generated fine air bubbles.

## Claims

1. A method for producing fructooligosaccharide, wherein, in a reaction where fructooligosaccharide is produced by reacting β-fructofuranosidase with sucrose and a reaction step where glucose by-produced by said reaction is decreased by glucose oxidase, an amount of dissolved oxygen is adjusted to 2 to 8 ppm.

2. A method for producing fructooligosaccharide, comprising the steps of: preparing a reaction mixture containing sucrose, β-fructofuranosidase, and glucose oxidase; and incubating the reaction mixture under optimum conditions for the β-fructofuranosidase, wherein, in the incubating step, an amount of dissolved oxygen is adjusted to 2 to 8 ppm.

3. The method according to claim 1 or 2, wherein the fructooligosaccharide is a sugar solution having a content ratio of fructooligosaccharide of 90% or more.

4. The method according to any one of claims 1 to 3, which is carried out by using a reaction vessel equipped with an aeration means and a stirring means.

5. The method according to claim 4, wherein the aeration means is a fine air bubble generating device.

6. The method according to any one of claims 1 to 5, wherein the amount of dissolved oxygen is adjusted to 2 to 6 ppm.
